# EUROPÄISCHE PATENTSCHRIFT

(11) **EP 3 551 144 B1**
(45) Veröffentlichungstag und Bekanntmachung des Hinweises auf die Patenterteilung: **02.08.2023**
(21) Anmeldenummer: 17837861.8
(22) Anmeldetag: 07.12.2017
(51) Int. Cl.: A61F 9/02, G02B 27/01

(54) **APPARATUR ZUM STRAHLUNGSSCHUTZ DER AUGEN**
APPARATUS FOR PROTECTING EYES FROM RADIATION
APPAREIL POUR PROTÉGER LES YEUX D'UN RAYONNEMENT

(30) Priorität: 07.12.2016 DE 102016123634
(43) Veröffentlichungstag der Anmeldung: 16.10.2019
(73) Patentinhaber: Woermann, Bernd, 01328 Dresden (DE)
(72) Erfinder: Woermann, Bernd, 01328 Dresden (DE)
(74) Vertreter: Lippert Stachow Patentanwälte Rechtsanwälte Partnerschaft mbB
(86) Internationale Anmeldenummer: PCT/EP2017/081858
(87) Internationale Veröffentlichungsnummer: WO 2018/104465

(56) Entgegenhaltungen:
- CN-B- 102 573 720
- US-A1- 2011 214 082
- US-A1- 2014 333 773
- US-B1- 8 334 899
- US-B1- 8 588 448

## Beschreibung

Die Erfindung betrifft eine Apparatur zum Strahlungsschutz der Augen, insbesondere der Augenlinsen von medizinischem Personal, wie Diagnostiker, vor Röntgen- oder anderer ionisierender Strahlung während medizinischer Untersuchungen oder Behandlungen.

Es ist seit längerer Zeit bekannt, dass ein hohes Maß an Röntgenstrahlung eine Trübung der Augenlinse hervorrufen und grauen Star provozieren kann. Die ICRP (International Commission of Radiological Protection) hatte bereits eine Reduktion der zulässigen Strahlungsbelastung von 150 Millisievert auf 20 Millisievert als maximale Organdosis des Auges im Jahr vorgeschlagen.

Die derzeit verwendeten Schutzmaßnahmen für die Augen eines Diagnostikers sind Bleiglasbrillen, die richtig angewandt, einen gewissen Schutz bieten können. Bei Röntgenanlagen, insbesondere bei solchen mit mehreren Röntgenstrahlern, ist jedoch eine ausreichende Abschirmung schwer zu realisieren (Quelle: Prof. Dr. Dr. Reinhard Loose, "Strahlenschutz, damit nichts ins Auge geht", in "healthcare-in-europe.com", 29.05.2013) .

Auch wurde bisher versucht, die Ausdehnung der Strahlenfelder genau auszumessen und durch eine entsprechende Positionierung des Kopfes bzw. durch zusätzliche Abschirmungen mit Bleiglas eine Verringerung der Strahlungsbelastung für einen Diagnostiker zu erreichen.

Aus der EP 0 419 854 A1 geht beispielsweise eine Laserschutzbrille hervor, die einen direkten Blick auf den Einsatzort des Lasers erlaubt und die aus einer Brillenfassung in Form einer zum Gesicht des Brillenträgers offenen Schale ausgebildet ist, in deren Frontfläche für jedes Auge ein Schutzfilter eingesetzt ist. Seitlich der Brillenfassung befinden sich Schläfenplatten, die zusammen mit der Brillenfassung das Gesicht des Brillenträgers dicht umschließen und ein Eindringen von Strahlung hinter die Brillenfassung verhindert. Die Brillenfassung kann aus einem Leichtmetall, wie Aluminium oder einem geeigneten Kunststoff, bestehen.

Eine solche Laserschutzbrille lässt sich jedoch nicht auf eine Verwendung unter einer vergleichsweise sehr harten Röntgenstrahlung umrüsten, so dass ein hinreichender Strahlungsschutz nicht erreicht werden kann.

Ein ähnlicher Augenschutz vor UV-Strahlung bei Bräunungsgeräten geht aus der EP 1 488 768 A1 hervor, die eine für Strahlung zumindest partiell wenig durchlässige Einrichtung zum Abdecken des Auges oder beider Augen umfasst, die jedoch eine für Strahlung wenig durchlässige Öffnung umfasst, welche die Strahlung auf mindestens ein optisches Element passieren lässt, das optische Signale an das Auge weiterleitet. Das können auch eine LCD-Einrichtung, oder Spiegel zur Bildung von Mikroarrays, sein.

Eine solche Vorrichtung lässt sich neben dem Schutz des Benutzers vor künstlicher UV-Strahlung auch als Schutz vor Strahlung von Schweißgeräten verwenden. Ein hinreichender Schutz vor Röntgenstrahlung lässt sich mit einer solchen Vorrichtung ebenfalls nicht erreichen.

In der EP 0 780 783 A1 wird ein Virtual-Reality-Wiedergabesystem mit einer optischen Lageerkennungseinrichtung beschrieben, das aus einem Helm mit einem an der Vorderseite des Helms befindlichen optischen Lageererkennungssystem für das Virtual-Reality-Wiedergabesystem besteht.

Mit diesem Wiedergabesystem wird erreicht, dass sich das Scheinwelt-Bild, das vor dem Auge des Betrachters erzeugt wird, sinngemäß mit der Bewegung des Kopfes bewegt. Ein Schutz der Augen vor schädlicher Strahlung ist hier nicht vorgesehen und auch nicht notwendig.

Die US 8 334 899 B1, die als nächstliegender Stand der Technik betrachtet wird, ist eine Brillenkonstruktion und offenbart eine Betrachtungsvorrichtung mit Schutz vor Laserstrahlung mit einer Videokamera, die für die zu betrachtende Laserstrahlung sensitiv ist und mit einem oder mehreren Displays vor den Augen verbunden ist. Das System ermöglicht ein Verfahren zum Betrachten einer Laserstrahlung ohne Gefahr einer Schädigung der Augen. Das ist insbesondere von Vorteil, bei einer für das Auge unsichtbaren Laserstrahlung. Die Kamera kann Bestandteil der Brillenanordnung sein, oder am Kopf des Nutzers angeordnet sein.

Aus der US 8 588 448 B1 geht eine Brillenkonstruktion hervor, die eine freihändige Multimediakommunikation auch in störender Umgebung erlaubt, indem sich vor dem Brillenglas eine Anzeigeeinheit befindet, oder indem Informationen von oben in das Brillenglas eingespiegelt werden.

Die US 2011/214082 A1 zeigt ist eine ähnliche Brillenkonstruktion und bezieht sich auf das seitliche Einblenden von Informationen, wie Telefondaten, in ein durch ein Brillenglas sichtbares Bild.

Aus der CN 102 573 720 geht eine Schweißerhaube mit einem Schutzfenster und mit zwei Kameras vor der Haube und zwei Bildschirmen auf der Innenseite vor den Augen hervor.

Die US 2014/333775 B bezieht sich auf eine vor das Gesicht schwenkbare Audio/Video-Maske mit Oled-Displays auf der Innenseite. Auf der Vorderseite ist eine Kamera zur Echtzeitübertragung vorgesehen. Zusammenfassend kann festgestellt werden, dass aus dem Stand der Technik keine praktikablen Lösungen bekannt sind, die ein Arbeiten unter Röntgenstrahlung mit vollständigem Augenschutz bei gleichzeitiger Betrachtung des der Röntgenstrahlung ausgesetzten Objektes durch einen Diagnostiker erlauben würden.

Hier setzt die Erfindung an, indem es Aufgabe der Erfindung ist, eine Apparatur zum Strahlungsschutz der Augen von medizinischem Personal während medizinischer Untersuchungen oder Behandlungen zu schaffen, die einerseits leicht zu handhaben ist und die andererseits eine Abschirmung der Augen vor Röntgenstrahlung oder anderer ionisierender Strahlung um bis zu 100% gewährleistet, die aber dennoch beste Arbeits- und Sichtbedingungen für den Träger der Apparatur, wie einem Diagnostiker, bietet.

Erreicht wird das einer Apparatur mit den Merkmalen des Anspruchs 1.

Mit dieser Apparatur kann vor den Augen des Trägers ein dynamisches verzögerungsfreies Bild erzeugt werden, das dem realen Abbild einer Szene vor dem Auge (zumindest weitgehend) entspricht, wobei ein räumliches Sehen problemlos möglich ist.

Die Abschirmung besteht bevorzugt aus Blei, wie einer Bleiplatte, kann aber auch aus einem anderen geeigneten Material bestehen, sofern die gewünschte Abschirmung gewährleistet ist.

In einer Fortführung der Erfindung umfasst die Abschirmung den seitlichen Schläfenbereich, so dass ein noch besserer Strahlungsschutz für die Augen erreicht wird, insbesondere wenn der Träger der Apparatur seinen Kopf zur Seite gedreht hat.

In einer Fortführung der Erfindung besteht die Einrichtung zur stereoskopischen Widergabe von Videoinformationen aus jeweils einem vor jedem Auge angeordneten Bildschirm innerhalb des Gehäuses.

Die Einrichtung zur stereoskopischen Widergabe von Videoinformationen kann auch aus einem einzigen vor beiden Augen des Trägers befindlichen und ggf. runden Bildschirm bestehen, oder der Bildschirm ist ein Split-Screen-Bildschirm, der für einen Stereoeindruck für jedes Auge ein separates Bild erzeugt.

Alternativ kann der Bildschirm mit einem Shutter gekoppelt sein, der die Bilder für beide Augen nacheinander erzeugt, so dass für den Träger der Apparatur ein Stereoeindruck entsteht.

In jedem Fall wird dem Träger ein realistisches stereoskopisches Abbild der Vorgänge vor der Apparatur vermittelt, welches dem medizinischen Personal ein sicheres und für die Augen gefahrloses Arbeiten ermöglicht.

Um den relativ geringen Abstand zwischen Bildschirm und Auge auszugleichen, ist zwischen den Augen des Trägers und dem Bildschirm oder den Bildschirmen jeweils eine Optik zur Fokussierung des angezeigten Bildes auf die Augen angeordnet. Dadurch wird ein ermüdungsfreies Betrachten des Bildschirmes ermöglicht.

In einer weiteren Ausgestaltung der Erfindung ist das optischen Aufnahmegerät bzw. die Kamera eine stereoskopische Kamera, die unmittelbar vor der Abschirmung positioniert ist.

Alternativ können vor der Abschirmung zwei in einem Abstand zueinander und nebeneinander angeordnete Einzelkameras vorgesehen sein, deren Abstand zueinander auf den Augenabstand einstellbar ist. Der stereoskopische Eindruck beim Träger der Apparatur kann dadurch auf die individuellen Gegebenheiten angepasst, bzw. optimiert werden.

Weiterhin sind die optischen Achsen der Einzelbildschirme mechanisch durch Verschieben, oder elektronisch durch Verschieben der Abbildungen auf den Einzelbildschirmen, auf den jeweiligen Augenabstand des Trägers einstellbar, so dass beide Einzelbilder durch das Gehirn zu einem einzigen Bild verschmolzen werden können.

Um ein störlichtfreies und kontrastreiches Betrachten des Bildschirmes oder der Bildschirme zu gewährleisten, liegt die schalenartige Fassung im Wesentlichen lichtdicht, die Augen des Trägers einschließend, am Kopf an.

Zum Schutz vor Beschädigung und Verschmutzung ist vor der stereoskopischen Kamera bzw. den Einzelkameras eine glasklar durchsichtige und die Fassung abschließende Schutzscheibe angeordnet, die mit der schalenartigen Fassung lösbar verbunden ist.

Um eine individuelle Anpassung an den Kopf des Trägers zu ermöglichen, sind der Ring über einen nicht dargestellten Rastmechanismus im Umfang einstellbar und das Kopfpolster am Ring verschwenkbar gelagert.

Schließlich sind die Nasenauflage und der Ring, zumindest im Kontaktbereich mit dem Kopf, mit einer Polsterung versehen.

Im Interesse einer möglichst leichten und somit ermüdungsfrei zu tragenden Apparatur bestehen sämtliche Bauteile des Gehäuses, wie die Fassung, die Optik und die Schutzscheibe, außer der Abschirmung und der Videoaufnahme- und Wiedergabeeinrichtungen mit zugehöriger Elektronik, aus einem möglichst leichten Kunststoff. Das Gewicht der Apparatur wird dann hauptsächlich durch das Gewicht der Abschirmung bestimmt.

Das Kopfpolster kann zumindest teilweise zum Gewichtsausgleich für das mit dem Ring verbundene Gehäuse genutzt werden, so dass eine weitgehend gleichmäßige Druckbelastung des Kopfes durch den Ring erreicht wird. Um das zu erreichen, könnte das Kopfpolster zugleich als Batteriefach gestaltet werden, so dass jegliche Kabelverbindung zwischen der Apparatur und externen Geräten entfallen kann.

Der besondere Vorteil der erfindungsgemäßen Apparatur ist darin zu sehen, dass die Augenpartie in Richtung der Quelle der Röntgenstrahlung vollständig mit einem undurchsichtigen, strahlungsundurchlässigen Material abgeschirmt ist, wobei die Szene vor der Apparatur in Echtzeit von der Einrichtung zur stereoskopischen Wiedergabe von Videoinformationen auf der Rückseite der Abschirmung wiedergegeben wird.

Über den Bildschirm oder die Bildschirme können auch Zusatzinformationen, wie Blutdruck oder andere Werte, sowie Bildinformationen aus anderen Untersuchungen oder Vergleichswerte oder -bilder, etc. eingeblendet werden.

Als für Röntgenstrahlung undurchlässiges Material kommt eine die Augenpartie vollkommen abdeckende Bleiplatte mit geringst möglichem Gewicht, oder ein anderes Abschirmungsmaterial für Röntgenstrahlung in Betracht, wobei für die Auswahl des strahlungsundurchlässigen Materials wesentlich ist, dass die die Abschirmung durchdringende Strahlungsdosis auf Werte nahe Null reduziert wird.

Weiterhin können auch die Seitenpartien in den Strahlungsschutz mit einem strahlungsundurchlässigen Material einbezogen sein.

Schließlich ist es im Interesse einer ermüdungsfreien Arbeit von Vorteil, wenn das Gehäuse, oder zumindest die schalenartige Fassung auf der Innenseite eine entspiegelte oder matte Oberfläche aufweist.

Die Erfindung wird nachfolgend an einem Ausführungsbeispiel näher erläutert. In den zugehörigen Zeichnungen zeigen:

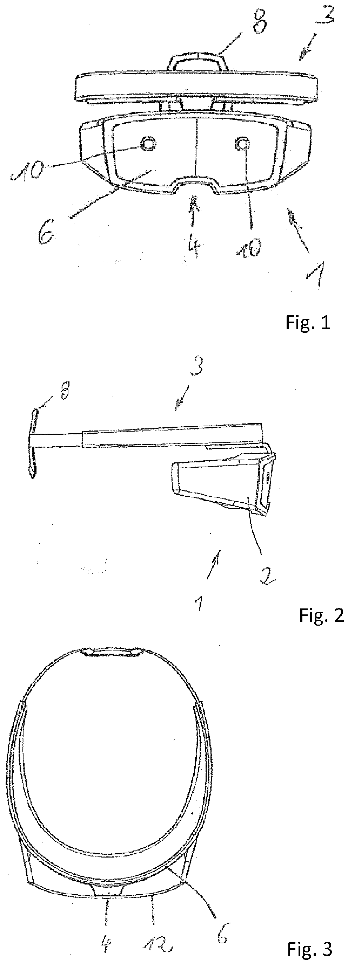

- Fig. 1:: eine Vorderansicht der erfindungsgemäßen Apparatur zum Strahlungsschutz der Augen;
- Fig. 2:: eine Seitenansicht der Apparatur nach Fig. 1;
- Fig. 3:: eine Draufsicht auf die Apparatur nach Fig. 1;

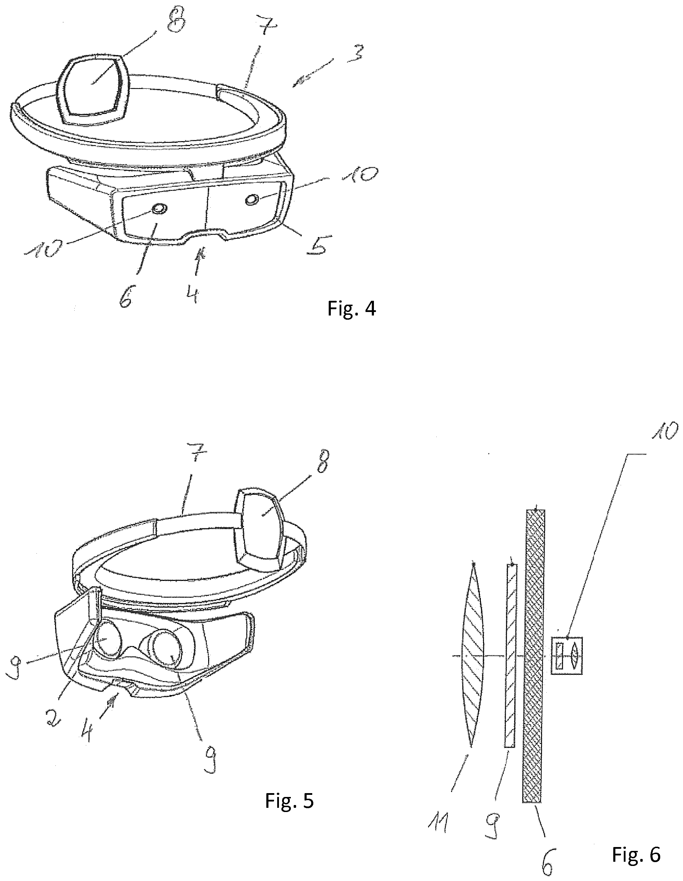

- Fig. 4:: eine perspektivische Darstellung der Apparatur nach Fig. 1;
- Fig. 5:: eine perspektivische Rückansicht der Apparatur nach Fig. 1; und
- Fig. 6:: eine schematische Darstellung der Einrichtung zur stereoskopischen Wiedergabe von Videoinformationen, sowie eines optischen Aufnahmegerätes bzw. einer Kamera vor einer Abschirmung.

Die Apparatur zum Strahlungsschutz der Augen umfasst ein Gehäuse 1, das aus einer schalenartigen Fassung 2 besteht, mit einer offenen Rückseite, welche den Kopf einschließlich der Augenpartie und seitlich die Schläfen des Trägers lichtdicht umschließt. Weiterhin ist die schalenartige Fassung 2 an einer Trageeinrichtung 3 zum Aufsetzen auf dem Kopf befestigt und mit einer Nasenauflage 4 zum Positionieren und Abstützen der schalenartigen Fassung 3 auf dem Nasenrücken des Trägers versehen.

Im vorderen Bereich der schalenartigen Fassung 2, also auf der dem Kopf abgewandten Seite, befindet sich eine im Wesentlichen über deren gesamte Breite reichende frontseitige Öffnung 5 zur Aufnahme einer Abschirmung 6 aus einem für Röntgen- oder ionisierende Strahlung undurchlässigen Material. Diese Abschirmung 5, die leicht bogenförmig verläuft, reduziert die die Augen erreichende Strahlungsdosis von einer Quelle für Röntgenstrahlung auf Werte nahe Null, insbesondere dann, wenn die Abschirmung 6 aus einer Bleiplatte besteht.

Die Abschirmung 6 kann an Stelle aus Blei, auch aus einem anderen geeigneten Material gefertigt werden, sofern die gewünschte Abschirmung gewährleistet ist. So dass dadurch eine Gewichtsreduzierung erreicht werden kann.

Als Abschirmung 6 kommt insbesondere eine mindestens die Augenpartie vollkommen abdeckende Bleiplatte mit geringst möglichem Gewicht, oder ein anderes Abschirmungsmaterial für Röntgenstrahlung in Betracht, wobei für die Auswahl des strahlungsundurchlässigen Materials wesentlich ist, dass die das Auge erreichende Strahlungsdosis auf Werte nahe Null reduziert wird.

Die Abschirmung 5 kann auch den seitlichen Schläfenbereich umfassen, so dass ein noch besserer Strahlungsschutz für die Augen erreicht wird, was insbesondere dann von Vorteil ist, wenn der Träger der Apparatur seinen Kopf zur Seite gedreht hat, so dass die frontseitige Abschirmung 5 nicht mehr in Richtung der Quelle für die Röntgenstrahlung weist. Durch die zusätzliche seitliche Abschirmung kann die Röntgenstrahlung auch nicht ungewollt von der Seite in die Augen einstrahlen.

Alternativ kann die schalenartige Fassung 2 auch insgesamt aus einem für Röntgenstrahlung undurchlässigem oder kaum durchlässigen Material gefertigt werden.

Die Trageeinrichtung 3 für das Gehäuse 1 ist ein der Kopfform des Trägers angepasster bzw. sich selbst anpassender Ring 7, an dem das Gehäuse 1 mit der schalenartigen Fassung 2 hängend befestigt ist. Um eine individuelle Anpassung des Ringes 7 an den Kopf des Trägers zu ermöglichen, ist dieser über einen nicht dargestellten Rastmechanismus, o.dgl. im Umfang einstellbar.

Weiterhin befindet sich am Ring 7, diametral gegenüberliegend zum Gehäuse 1, ein bevorzugt schwenkbar gelagertes Kopfpolster 8, das sich am Hinterkopf abstützt und ein sicheres Tragen der Apparatur ermöglicht.

Das Kopfpolster 8 kann auch zumindest teilweise als ein Gegengewicht für das mit dem Ring 7 verbundene Gehäuse 1 verwendet werden. Beispielsweise könnte zu diesem Zweck im Kopfpolster 8 ein Batteriefach untergebracht sein, so dass eine gleichmäßigere Druckbelastung des Kopfes durch den Ring 7 erreicht wird.

Zur besseren Lastverteilung können auch ein oder mehrere am Ring 7 befestigte Tragbänder vorgesehen sein, die sich beim Aufsetzen der Apparatur von oben am Kopf des Trägers anlegen.

Ergänzend können die Nasenauflage 4 und der Ring 7 zumindest im Kontaktbereich mit dem Kopf, also den Auflagestellen, mit einer Polsterung versehen sein.

Auf der den Augen des Trägers zugewandten Rückseite der Abschirmung 6 befindet sich wenigstens eine Wiedergabeeinrichtung 9 zur stereoskopischen Wiedergabe von Videoinformationen, die mit einem optischen Aufnahmegerät 10 bzw. einer Kamera gekoppelt ist, die auf der den Augen des Trägers abgewandten Seite der Abschirmung 6 angeordnet ist, wobei die von dem Aufnahmegerät 10 aufgenommenen Bilder und Bildsequenzen unmittelbar und verzögerungsfrei auf die Wiedergabeeinrichtung 9 übertragen werden.

Diese Anordnung erlaubt es, vor den Augen des Trägers der Apparatur ein dynamisches Abbild zu erzeugen, das dem realen Abbild einer Szene vor dem Auge (zumindest weitgehend) entspricht, wobei ein räumliches Sehen problemlos möglich ist, wobei die die Apparatur erreichende Röntgenstrahlung die Augen des Trägers nicht erreichen kann, so dass ein gefahrloses Arbeiten möglich ist.

Wiedergabeeinrichtung 9 kann aus jeweils einem vor jedem Auge innerhalb der schalenartigen Fassung 2 positionierten Bildschirm bestehen, der ggf. auch einen runden Umriss haben kann.

Alternativ kann die Wiedergabeeinrichtung 9 auch aus einem einzigen vor beiden Augen des Trägers befindlichen Bildschirm bestehen, der mit einem Shutter gekoppelt ist, der die Bilder für beide Augen nacheinander erzeugt, so dass für den Träger der Apparatur ein Stereoeindruck entsteht.

Auch kann die Wiedergabeeinrichtung 9 ein Split-Screen-Bildschirm sein, der für einen Stereoeindruck für jedes Auge ein separates Bild erzeugt.

In jedem Fall wird dem Träger ein realistisches stereoskopisches Abbild der Vorgänge vor der Apparatur vermittelt, welches dem medizinischen Personal ein sicheres und für die Augen gefahrloses Arbeiten ermöglicht.

Um den relativ geringen Abstand zwischen Bildschirm und Auge auszugleichen, oder zum Ausgleich einer Fehlsichtigkeit, ist zwischen den Augen des Trägers und dem Bildschirm oder den Bildschirmen jeweils eine Optik 11 zur Fokussierung des angezeigten Bildes auf die Augen angeordnet, wodurch ein ermüdungsfreies Betrachten des Bildschirmes ermöglicht wird.

Weiterhin ist es im Interesse einer guten stereoskopischen Wiedergabe zweckmäßig, die optischen Achsen der Einzelbildschirme mechanisch durch Verschieben, oder elektronisch durch Verschieben der Abbildungen auf den Einzelbildschirmen, auf den jeweiligen Augenabstand des Trägers einzustellen.

Das optischen Aufnahmegerät 10 bzw. die Kamera kann eine stereoskopische Kamera sein, die unmittelbar vor der Abschirmung 6 positioniert ist.

Alternativ können vor der Abschirmung 6 auch zwei in einem Abstand zueinander und nebeneinander angeordnete Einzelkameras vorgesehen sein, deren Abstand zueinander auf den Augenabstand einstellbar sein kann. Damit kann der stereoskopische Eindruck beim Träger der Apparatur auf die individuellen Gegebenheiten angepasst werden.

Die elektrische Verbindung zwischen der Wiedergabeeinrichtung 9 und dem Aufnahmegerät 9 zur Stromversorgung und Informationsübertragung erfolgt mittels einer geeigneten Umverdrahtung, die um die Abschirmung 6 herumgelegt ist.

Um ein störlichtfreies Betrachten des Bildschirmes oder der Bildschirme zu gewährleisten, liegt die schalenartige Fassung 2 im Wesentlichen lichtdicht, die Augen des Trägers einschließend, am Kopf an.

Da auch Spiegelungen oder Lichtreflexe, verursacht durch die Wiedergabeeinrichtung 10, zu vermeiden, sollte das Gehäuse 1 oder zumindest die schalenartige Fassung auf der Innenseite eine entspiegelte oder matte Oberfläche aufweisen, was beispielsweise auch durch eine matte oder mattschwarze Beschichtung erreicht werden kann. Dadurch wird eine weitgehend ermüdungsfreie Benutzung der erfindungsgemäßen Apparatur durch einen Diagnostiker gewährleistet. Zum Schutz vor Beschädigung und Verschmutzung, sowie aus gestalterischen Grünen, ist vor der stereoskopischen Kamera bzw. den Einzelkameras eine glasklar durchsichtige und die schalenartige Fassung 2 nach vorn abschließende Schutzscheibe 12 angeordnet, die mit der schalenartigen Fassung 2 lösbar verbunden sein kann.

Der besondere Vorteil der erfindungsgemäßen Apparatur ist darin zu sehen, dass die Augenpartie mindestens in Richtung der Quelle der Röntgenstrahlung vollständig mit einem undurchsichtigen, strahlungsundurchlässigen Material abgeschirmt wird, wobei die die Szene vor der Apparatur in Echtzeit von der Wiedergabeeinrichtung 10 zur stereoskopischen Wiedergabe von Videoinformationen auf der Rückseite der Abschirmung 6 wiedergegeben wird.

Im Interesse einer möglichst leichten und somit ermüdungsfrei zu tragenden Apparatur bestehen sämtliche Bauteile des Gehäuses 1, wie die schalenartige Fassung 2, die Optik 11 und die Schutzscheibe 12, ausgenommen die Abschirmung und die Videoaufnahme- und Wiedergabeeinrichtungen mit zugehöriger Elektronik, aus einem möglichst leichten Kunststoffmaterial.

Über die Wiedergabeeinrichtung 10 können auch Zusatzinformationen wie Blutdruck oder andere Werte, sowie Bildinformationen aus anderen Untersuchungen, etc. eingeblendet werden.

### Bezugszeichenliste

- 1: Gehäuse
- 2: schalenartige Fassung
- 3: Trageeinrichtung
- 4: Nasenauflage
- 5: Öffnung
- 6: Abschirmung
- 7: Ring
- 8: Kopfpolster
- 9: Wiedergabeeinrichtung
- 10: Aufnahmegerät
- 11: Optik
- 12: Schutzscheibe

## Patentansprüche

1. Apparatur zum Strahlungsschutz der Augen, insbesondere der Augenlinsen, von medizinischem Personal, wie Ärzte oder Diagnostiker, vor Röntgen- oder anderer ionisierender Strahlung während medizinischer Untersuchungen, umfassend ein vor der Augenpartie am Kopf positionierbares Gehäuse,
wobei das Gehäuse (1) aus einer die Augenpartie und seitlich die Schläfen des Trägers lichtdicht umschließenden schalenartigen Fassung (2) besteht,
wobei die schalenartige Fassung (2) einerseits mit einer Trageeinrichtung (3) zum Aufsetzen auf dem Kopf und andererseits mit einer Nasenauflage (4) zum Positionieren auf dem Nasenrücken des Trägers versehen ist,
wobei die Trageeinrichtung (3) als ein der Kopfform angepasster bzw. sich selbst anpassender Ring (7) ausgebildet ist, an dem das Gehäuse (1) mit der schalenartigen Fassung (2) hängend befestigt ist und dass am Ring (7) diametral gegenüberliegend zum Gehäuse (1) ein Kopfpolster (8) angeordnet ist, das sich am Hinterkopf abstützt,
wobei die schalenartige Fassung (2) mit einer über dessen gesamte Breite reichenden frontseitigen Öffnung (5) versehen ist, in die eine Abschirmung (6) aus einem für Röntgen- oder ionisierende Strahlung undurchlässigen Material eingepasst ist, und
wobei auf der den Augen zugewandten Rückseite der Abschirmung (6) innerhalb der schalenförmigen Fassung (2) wenigstens eine Wiedergabeeinrichtung (9) zur stereoskopischen Wiedergabe von Videoinformationen in Echtzeit angeordnet ist, die mit einem optischen Aufnahmegerät bzw. einer Kamera (10) gekoppelt ist, die auf der den Augen des Trägers abgewandten Seite der Abschirmung (6) vor dieser angeordnet ist.

2. Apparatur nach Anspruch 1, **dadurch gekennzeichnet, dass** die Abschirmung (6) aus Blei oder einem anderen geeignetem Material besteht.

3. Apparatur nach Anspruch 1 und 2, **dadurch gekennzeichnet, dass** die Abschirmung den seitlichen Schläfenbereich des Kopfes umfasst.

4. Apparatur nach Anspruch 1, **dadurch gekennzeichnet, dass** die Wiedergabeeinrichtung (9) zur stereoskopischen Wiedergabe von Videoinformationen aus jeweils einem vor jedem Auge angeordneten Bildschirm besteht.

5. Apparatur nach Anspruch 1, **dadurch gekennzeichnet, dass** die Wiedergabeeinrichtung (9) zur stereoskopischen Widergabe von Videoinformationen aus einem einzigen vor beiden Augen des Trägers positionierten Bildschirm besteht.

6. Apparatur nach Anspruch 5, **dadurch gekennzeichnet, dass** der Bildschirm ein Split-Screen-Bildschirm ist, der für jedes Auge für einen Stereoeindruck ein separates Bild erzeugt.

7. Apparatur nach Anspruch 5, **dadurch gekennzeichnet, dass** der Bildschirm mit einem Shutter gekoppelt ist, der die Bilder für beide Augen nacheinander erzeugt, so dass ein Stereoeindruck entsteht.

8. Apparatur nach einem der Ansprüche 4 bis 7, **dadurch gekennzeichnet, dass** zwischen den Augen des Trägers und dem Bildschirm oder den Bildschirmen jeweils eine Optik (11) zur Fokussierung des angezeigten Bildes auf die Augen angeordnet ist.

9. Apparatur nach einem der Ansprüche 1 bis 8, **dadurch gekennzeichnet, dass** das optische Aufnahmegerät bzw. die Kamera (10) eine stereoskopische Kamera ist, die unmittelbar vor der Abschirmung (6) angeordnet ist.

10. Apparatur nach einem der Ansprüche 1 bis 8, **dadurch gekennzeichnet, dass** vor der Abschirmung (6) zwei in einem Abstand zueinander und nebeneinander angeordnete Einzelkameras vorgesehen sind, deren Abstand zueinander auf den Augenabstand einstellbar ist.

11. Apparatur nach einem der Ansprüche 1 bis 10, **dadurch gekennzeichnet, dass** die optischen Achsen der Einzelbildschirme mechanisch durch Verschieben der Einzelbildschirme, oder elektronisch durch Verschieben der Abbilder auf den jeweiligen Augenabstand des Trägers einstellbar sind.

12. Apparatur nach einem der Ansprüche 1 bis 11, **dadurch gekennzeichnet, dass** schalenförmige die Fassung (2) im Wesentlichen lichtdicht, die Augen des Trägers einschließend, am Kopf anliegt.

13. Apparatur nach einem der Ansprüche 1 bis 12, **dadurch gekennzeichnet, dass** vor der stereoskopischen Kamera bzw. den Einzelkameras eine glasklar durchsichtige und die schalenförmige Fassung (2) abschließende Schutzscheibe (12) angeordnet ist.

14. Apparatur nach Anspruch 11, **dadurch gekennzeichnet, dass** der Ring (7) über einen nicht dargestellten Rastmechanismus im Umfang einstellbar ist.

15. Apparatur nach Anspruch 11, **dadurch gekennzeichnet, dass** das Kopfpolster (8) am Ring (7) verschwenkbar gelagert ist.

16. Apparatur nach einem der Ansprüche 1 bis 15, **dadurch gekennzeichnet, dass** die Nasenauflage (4) und der Ring (7) zumindest im Kontaktbereich mit dem Kopf mit einer Polsterung versehen sind.

17. Apparatur nach einem der Ansprüche 1 bis 16, **dadurch gekennzeichnet, dass** sämtliche Bauteile, wie das Gehäuse (1), die schalenartige Fassung (2), die Optik (11) und die Schutzscheibe (12), außer der Abschirmung (6) und der Videoaufnahme- und Wiedergabeeinrichtungen mit zugehöriger Elektronik, aus Kunststoff bestehen.

18. Apparatur nach einem der Ansprüche 1 bis 17, **dadurch gekennzeichnet, dass** das Gehäuse (1), oder zumindest die schalenartige Fassung (2) auf der Innenseite eine entspiegelte oder matte Oberfläche aufweist.

## Claims

1. Radiation protection apparatus for the eyes, in particular the crystalline lenses, of medical staff such as physicians or diagnostic staff from x-ray radiation or other ionizing radiation during medical examinations, comprising a housing which is positionable on the head in front of the eye area,
wherein the housing (1) consists of a shell-like frame (2) which encloses the eye area and, to the sides, the temples of the wearer in lightproof fashion,
wherein the shell-like frame (2) is provided firstly with a wearing device (3) to be placed on the head and secondly with a nose support (4) to be positioned on the nasal bridge of the wearer,
wherein the wearing device (3) is in the form of a ring (7) which is adapted to the shape of the head or which independently adapts itself to the shape of the head, to which ring the housing (1) with the shell-like frame (2) is fastened in suspended fashion, and wherein a head cushion (8), which is supported on the back of the head, is arranged on the ring (7) diametrically opposite to the housing (1),
wherein the shell-like frame (2) is provided with a front-side opening (5) which ranges over the entire width thereof and fitted in which is a shield (6) made of a material that does not transmit x-ray or ionizing radiation, and
wherein at least one playback device (9) for stereoscopic playback of video information in real time is arranged within the shell-shaped frame (2) on the back side of the shield (6) facing the eyes, the said playback device being coupled to optical recording equipment or a camera (10) which is arranged in front of the shield (6) on the side thereof facing away from the eyes of the wearer.

2. Apparatus according to Claim 1, **characterized in that** the shield (6) consists of lead or any other suitable material.

3. Apparatus according to Claims 1 and 2, **characterized in that** the shield comprises the lateral temple region of the head.

4. Apparatus according to Claim 1, **characterized in that** the playback device (9) for stereoscopic playback of video information consists of a respective visual display unit arranged in front of each eye.

5. Apparatus according to Claim 1, **characterized in that** the playback device (9) for stereoscopic playback of video information consists of a single visual display unit positioned in front of both eyes of the wearer.

6. Apparatus according to Claim 5, **characterized in that** the visual display unit is a split-screen visual display unit which for the purpose of a stereo impression produces a separate image for each eye.

7. Apparatus according to Claim 5, **characterized in that** the visual display unit is coupled to a shutter which successively produces the images for the two eyes such that a stereo impression arises.

8. Apparatus according to any of Claims 4 to 7, **characterized in that** a respective optical unit (11) for focusing the displayed image onto the eyes is arranged between the eyes of the wearer and the visual display unit or units.

9. Apparatus according to any of Claims 1 to 8, **characterized in that** the optical recording equipment or the camera (10) is a stereoscopic camera arranged immediately in front of the shield (6).

10. Apparatus according to any of Claims 1 to 8, **characterized in that** two individual cameras arranged at a distance from and next to one another are provided in front of the shield (6), the distance between the said cameras being adjustable to the eye spacing.

11. Apparatus according to any of Claims 1 to 10, **characterized in that** the optical axes of the individual visual display units are adjustable to the respective eye spacing of the wearer, either mechanically by displacing the individual visual display units or electronically by displacing the images.

12. Apparatus according to any of Claims 1 to 11, **characterized in that** the shell-shaped frame (2) rests against the head in substantially lightproof fashion, in a manner to enclose the eyes of the wearer.

13. Apparatus according to any of Claims 1 to 12, **characterized in that** a crystal clear transparent protective pane (12) which completes the shell-shaped frame (2) is arranged in front of the stereoscopic camera or in front of the individual cameras.

14. Apparatus according to Claim 11, **characterized in that** the circumference of the ring (7) is adjustable by way of a latching mechanism not depicted here.

15. Apparatus according to Claim 11, **characterized in that** the head cushion (8) is pivotably mounted on the ring (7).

16. Apparatus according to any of Claims 1 to 15, **characterized in that** the nose support (4) and the ring (7) are provided with cushioning, at least in the contact area with the head.

17. Apparatus according to any of Claims 1 to 16, **characterized in that**, apart from the shield (6) and the video recording and playback devices and associated electronics, all component parts, such as the housing (1), the shell-like frame (2), the optical unit (11) and the protective pane (12), consist of plastic.

18. Apparatus according to any of Claims 1 to 17, **characterized in that** the housing (1), or at least the shell-like frame (2), has an anti-glare layer or matte surface on the inner side.

## Revendications

1. Appareil destiné à la radioprotection des yeux, notamment des cristallins, du personnel médical, comme les médecins ou diagnostiqueurs, contre les rayons X ou autres rayonnements ionisants lors d'examens médicaux, ledit appareil comprenant un boîtier qui peut être positionné sur la tête devant la zone située autour des yeux,
le boîtier (1) comprenant une monture (2) en forme de coque qui enferme la zone située autour des yeux et latéralement les tempes du porteur de manière étanche à la lumière,
la monture (2) en forme de coque étant pourvue d'une part d'un dispositif de port (3) destiné à être placé sur la tête et d'autre part d'une plaquette nasale (4) destinée à être positionnée sur l'arête nasale du porteur,
le dispositif de port (3) étant conçu comme un anneau (7) qui est adapté ou qui s'adapte automatiquement à la forme de la tête et sur lequel le boîtier (1) pourvu de la monture (2) en forme de coque est fixé de manière suspendue et un coussinet de tête (8), qui s'appuie sur l'arrière de la tête, étant disposé sur l'anneau (7) diamétralement à l'opposé par rapport au boîtier (1),
la monture (2) en forme de coque étant pourvue d'une ouverture frontale (5) qui s'étendant sur toute sa largeur et dans laquelle un blindage (6) en un matériau opaque aux rayons X ou aux rayonnements ionisants est inséré de manière ajustée, et
au moins un dispositif de reproduction (9) destiné à a reproduction stéréoscopique d'informations vidéo en temps réel, qui est couplé à un appareil de capture optique ou à une caméra (10), qui est disposée devant ledit blindage (6) sur le côté de celui-ci qui est à l'opposé des yeux du porteur, étant disposé à l'intérieur de la monture (2) en forme de coque du côté arrière du blindage (6) qui est dirigé vers les yeux.

2. Appareil selon la revendication 1, **caractérisé en ce que** le blindage (6) est en plomb ou en un autre matériau approprié.

3. Appareil selon les revendications 1 et 2, **caractérisé en ce que** le blindage comprend la zone temporale latérale de la tête.

4. Appareil selon la revendication 1, **caractérisé en ce que** le dispositif de reproduction (9) destiné à la reproduction stéréoscopique d'informations vidéo comprend un blindage disposé devant chaque oeil.

5. Appareil selon la revendication 1, **caractérisé en ce que** le dispositif de reproduction (9) destiné à la reproduction stéréoscopique d'informations vidéo comprend un écran unique positionné devant les deux yeux du porteur.

6. Appareil selon la revendication 5, **caractérisé en ce que** l'écran est un écran de type Split Screen qui génère une image séparée pour chaque oeil pour une impression stéréo.

7. Appareil selon la revendication 5, **caractérisé en ce que** l'écran est couplé à un obturateur qui génère successivement les images pour les deux yeux de façon à créer une impression stéréo.

8. Appareil selon l'une des revendications 4 à 7, **caractérisé en ce qu'**une optique (11) de focalisation de l'image affichée est disposée sur les yeux entre les yeux du porteur et l'écran ou chacun des écrans.

9. Appareil selon l'une des revendications 1 à 8, **caractérisé en ce que** l'appareil de capture optique ou la caméra (10) est une caméra stéréoscopique qui est disposée directement devant le blindage (6).

10. Appareil selon l'une des revendications 1 à 8, **caractérisé en ce que** deux caméras individuelles qui sont disposées à distance l'une de l'autre et à côté l'une de l'autre et dont la distance entre elles peut être ajustée à la distance entre les yeux, sont prévues devant le blindage (6).

11. Appareil selon l'une des revendications 1 à 10, **caractérisé en ce que** les axes optiques des écrans individuels peuvent être ajustés mécaniquement par coulissement des écrans individuels ou électroniquement par coulissement les images reproduites à la distance respective entre les yeux du porteur.

12. Appareil selon l'une des revendications 1 à 11, **caractérisé en ce que** la monture (2) en forme de coque vient en appui sur la tête de manière sensiblement étanche à la lumière, en enfermant les yeux du porteur.

13. Appareil selon l'une des revendications 1 à 12, **caractérisé en ce qu'**une vitre de protection (12) transparente comme du verre et obturant la monture (2) en forme de coque est disposée devant la caméra stéréoscopique ou les caméras individuelles.

14. Appareil selon la revendication 11, **caractérisé en ce que** l'anneau (7) peut être ajusté en taille par le biais d'un mécanisme à encliquetage non représenté.

15. Appareil selon la revendication 11, **caractérisé en ce que** le coussin de tête (8) est monté de manière pivotante sur la bague (7).

16. Appareil selon l'une des revendications 1 à 15, **caractérisé en ce que** la plaquette nasale (4) et l'anneau (7) sont munis d'un rembourrage au moins dans la zone de contact avec la tête.

17. Appareil selon l'une des revendications 1 à 16, **caractérisé en ce que** tous les composants, comme le boîtier (1), la monture (2) en forme de coque, l'optique (11) et la vitre de protection (12), à l'exception du blindage (6) et des dispositifs de capture vidéo et de reproduction avec l'électronique associée, sont en matière synthétique.

18. Appareil selon l'une des revendications 1 à 17, **caractérisé en ce que** le boîtier (1) ou au moins la monture (2) en forme de coque comporte du côté intérieur une surface antireflet ou mate.
